# EUROPÄISCHE PATENTSCHRIFT

(11) **EP 2 396 021 B1**
(45) Veröffentlichungstag und Bekanntmachung des Hinweises auf die Patenterteilung: **19.08.2015**
(21) Anmeldenummer: 10723482.5
(22) Anmeldetag: 16.02.2010
(51) Int. Cl.: A61K 38/16, A61K 39/395, A61P 29/00, A61P 35/00

(54) **IN VITRO VERWENDUNG VON NOTCH-REGULATOREN ZUR MODULATION DER IMMUNANTWORT DURCH INDUKTION/SUPPRESSION VON INTERLEUKIN-22**
IN VITRO USE OF NOTCH REGULATORS FOR MODULATING THE IMMUNE RESPONSE BY INDUCTION/SUPPRESSION OF INTERLEUKIN-22
UTILISATION IN VITRO DE RÉGULATEURS NOTCH POUR MODULER LA RÉPONSE IMMUNITAIRE PAR INDUCTION/SUPPRESSION D'INTERLEUKINE-22

(30) Priorität: 16.02.2009 DE 102009009603
(43) Veröffentlichungstag der Anmeldung: 21.12.2011
(73) Patentinhaber: Miltenyi Biotec GmbH, 51429 Bergisch Gladbach (DE)
(72) Erfinder: SCHEFFOLD, Alexander, 51069 Köln (DE); RUTZ, Sascha, 10117 Berlin (DE); HEINRICH, Frederick, 10117 Berlin (DE)
(74) Vertreter: Lange, Sven
(86) Internationale Anmeldenummer: PCT/DE2010/000212
(87) Internationale Veröffentlichungsnummer: WO 2010/091679

(56) Entgegenhaltungen:
- WO-A1-98/20142
- WO-A1-03/011317
- WO-A2-02/096952
- WO-A2-2008/092445
- ANASTASI EMANUELA ET AL: "Expression of activated Notch3 in transgenic mice enhances generation of T regulatory cells and protects against experimental autoimmune diabetes" JOURNAL OF IMMUNOLOGY, AMERICAN ASSOCIATION OF IMMUNOLOGISTS, US, Bd. 171, Nr. 9, 1. November 2003 (2003-11-01), Seiten 4504-4511, XP002486581 ISSN: 0022-1767

## Beschreibung

Die Erfindung betrifft ein in vitro Verfahren zur gezielten Modulation der Interleukin-22 Expression von T-Zellen durch Beeinflussung des Notch-Signalweges. Die Erfindung beinhaltet:
1. die Möglichkeit zur Generierung von Interleukin(IL)-22-produzierenden T-Zellen, durch die Aktivierung des Notch-Signalweges;
2. die Möglichkeit der Inhibition der IL-22 Expression von T-Zellen durch die Inhibition des Notch-Signalweges.

Den vielfältigen Aufgaben von T-Helfer-Zellen entsprechend, gibt es unterschiedliche Subpopulationen dieser Zellen. Sie zeichnen sich durch die differentielle Expression von löslichen Botenstoffen, den Zytokinen, aus.

Eine Subpopulation von T-Helfer-Zellen, die sogenannten Th1-Zellen, exprimieren Zytokine, die die zelluläre Immunantwort dirigieren können. Hierzu gehören Interferon(IFN)-gamma und Tumor-Nekrose-Faktor (TNF)-alpha und -beta. Th2-Zellen sind notwendig für humorale Immunantworten. Zu den Th2-Zytokinen zählen IL-4, IL-5, IL-10 und IL-13. Eine weitere Subpopulation von T-Helfer-Zellen, die sogenannten Th17-Zellen, sind gekennzeichnet durch die Produktion des Zytokins IL-17 und wirken ebenfalls pro-inflammatorisch.
IL-22 wirkt nicht auf Leukozyten, sondern auf Hepatozyten, Keratinozyten oder auch Darmepithelzellen. IL-22 ist sowohl an Entzündungsprozessen als auch an der Gewebereparatur und Wundheilung beteiligt.

IL-22 induziert pro-inflammatorische Antworten durch die Induktion von Zytokinen, Chemokinen und Akute-Phase-Proteinen in vielen Zelltypen. Darüber hinaus induziert IL-22 die Produktion von antimikrobiellen Peptiden, wie b-Defensinen und S100 Proteinen. Es kann eine starke antimikrobielle Reaktion in vitro induzieren und ist mit verschiedenen viralen und bakteriellen Infektionen assoziiert.

Die Funktion von IL-22 im Entzündungsgeschehen hängt entscheidend vom Zielgewebe ab. Obwohl einige Berichte über eine pro-inflammatorische pathogene Funktion von IL-22 vor allem in Entzündungsreaktionen der Haut existieren (IL-22 ist in psoriatischen Läsionen überexprimiert), wird in anderen Systemen eine protektive Wirkung gefunden. IL-22 wirkt bei Hepatitis oder ulzerierender Colitis protektiv.

IL-22 wird bisher vorwiegend als Th17-Zytokin beschrieben. IL-22 wird jedoch auch von NK-Zellen und von dendritischen Zellen exprimiert.

In vitro kann IL-22 in Th-Zellen durch IL-23 und IL-6 induziert werden, während TGF-b inhibierend wirkt. In vivo ist vor allem IL-23 für die IL-22 Expression notwendig. Die molekularen Mechanismen sind bisher nicht bekannt.

Da IL-22 in vielen Prozessen - unter anderem im Krankheitsverlauf von unterschiedlichen Erkrankungen - eine Rolle spielt, war es die Aufgabe der Erfindung, die IL-22 Produktion in T-Zellen zu regulieren, insbesondere zu verstärken oder abzuschalten.

Die Aufgabe konnte durch die Ausführungsformen der Ansprüche realisiert werden.

In einer ersten Ausführungsform betrifft die Erfindung eine in vitro Verwendung von Notch-Regulatoren, ausgewählt aus der Gruppe umfassend Induktoren der signalaktiven Notch-Moleküle und/oder Notch-Inhibitoren, zur Modifikation der Interleukin-22-Produktion in T-Zellen.

Es war völlig überraschend, dass über die Regulation des Notch-Signalwegs die Produktion von IL-22 in T-Zellen moduliert werden kann.

Außerdem bevorzugt ist die Verwendung, dadurch gekennzeichnet, dass die T-Zellen ausgewählt sind aus der Gruppe umfassend naiven T-Zellen, Gedächtnis-T-Zellen, proinflammatorischen Th1-Zellen, proinflammatorische Th2-Zellen und proinflammatorische Th17-Zellen. Es war völlig überraschend, dass die Regulation des Notch-Signalwegs in all diesen Zellen zur Modulation der IL-22 Produktion führt.

Notch ist in einer latenten Form immer in der Zelle vorhanden. Diese latente Form wird durch Aktivatoren in eine (signal)aktive (signalaktiv = aktiv) Form überführt. Dies geschieht im Falle des Notch-Rezeptors durch proteolytische Spaltung. Dies bedeutet gleichzeitig, dass die aktive Form eine andere Struktur/Beschaffenheit besitzt als die latente Form.

Dadurch kann eine signalaktive Form auf zwei Wegen in die Zelle gebracht werden
a) Durch physiologische Aktivierung der Rezeptoren: Aktivatoren für Notch sind nach dieser Definition die Notch-Liganden, Notchligand-Protein, Notchligand-Fusionsproteinen, Notchligand-Fragmente, Notchligand-exprimierenden Zellen und/oder stimulierende anti-Notch-Antikörper.
b) Durch direkte Transfektion der Zellen mit der konstitutiv aktiven Form von Notch (Notch-intracellular domain (NICD)), bevorzugt Notch-Rezeptor-1, -2, -3 oder -4.

Bevorzugt sind die Induktoren der signalaktiven Notch-Moleküle ausgewählt aus der Gruppe umfassend Aktivatoren für Notch, bevorzugt Notch-Liganden, Notchligand-Protein, Notchligand-Fusionsproteinen, Notchligand-Fragmente, Notchligand-exprimierenden Zellen und/oder stimulierende anti-Notch-Antikörper und/oder Notch-Rezeptoren, bevorzugt Notch-Rezeptor-1, -2, -3 oder -4.

Außerdem vorteilhaft ist die Verwendung endogener Notch-Moleküle. Endogene Notch-Moleküle haben den Vorteil, dass sie nur spezifisch auf eine Zelle wirken. Dadurch ist eine exakte Regulation möglich.

Die Notch-Rezeptoren sind bevorzugt ausgewählt aus der Gruppe umfassend Notch-1, -2, -3 und/oder -4.

Die Notch-Liganden sind bevorzugt ausgewählt aus der Gruppe umfassend die Jagged-Familie, insbesondere Jagged-1 und/oder -2 und/oder Delta-like-Familie, insbesondere Delta-like-1, -2, und/oder -4.

Es war dabei überraschend, dass die Notch-Liganden aus der Delta-like Familie, insbesondere Delta-like4, die IL-22 Induktion bewirken.

Es war auch überraschend, dass pDC, insbesondere nach Stimulation mit Liganden der Toll-like-Rezeptoren, bevorzugt TLR 1-13 und insbesondere bevorzugt TLR9, besonders große Mengen an Delta-like 4 exprimieren und unter diesen Bedingungen besonders effizient die Induktion von IL-22 in T-Zellen bewirken.

Notch-Ligand-Fragmente bzw. Protein-Fragmente sind bevorzugt solche, wie in der WO 2004/024764 A1 offenbart. Die dort offenbarten Konstrukte und Fragmente der Notch-Liganden können entweder für eine Aktivierung oder Inhibition des Notch-Signalweges eingesetzt werden. Bevorzugt weisen die Fragmente im wesentlichen die gleiche Aktivität wie das Molekül auf, aus dem sie gewonnen wurden.

Bevorzugt sind die Inhibitoren der Notch-Aktivierung, ausgewählt aus der Gruppe umfassend rekombinante Proteine, Proteinfragmente und/oder Peptide von Notch oder Notchliganden, pharmakologischen Inhibitoren bevorzugt gamma-Sekretase-Inhibitoren, Antikörpern gegen Notch-Moleküle zur Hemmung der Induktion und/oder Expression von Interleukin-22.

In eine bevorzugten Ausführungsform betrifft die Erfindung die in vitro Verwendung von Induktoren der signalaktiven Notch-Moleküle und/oder signalaktive Notch-Molekülen zur Generierung von Interleukin-22-produzierenden T-Zellen.

Es war überraschend, dass T-Zellen, wenn sie mit signalaktivem Notch in Kontakt gebracht werden, IL-22 exprimieren.

Außerdem bevorzugt ist die in vitro Verwendung von Induktoren der signalaktiven Notch-Moleküle.und/oder signalaktive Notch-Molekülen zur Induktion und/oder Expression von Interleukin-22.

Besonders bevorzugt ist, dass die T-Zellen mit signalaktiven Notch-Molekülen in Kontakt gebracht werden. Durch die Aktivierung von Notch wird die IL-22 Produktion der T-Zellen induziert.

Außerdem bevorzugt ist die Verwendung, wobei signalaktives Notch-1, -2, -3 und/oder -4 überexprimiert wird. Diese Verwendung führt zu einer besonders schnellen Erhöhung der IL-22 Produktion. Dem Fachmann sind Methoden bekannt, um die Überexpression von bestimmten Proteinen zu induzieren sowie signalaktive Varianten von Proteinen herzustellen.

Außerdem vorteilhaft ist das In-Kontakt-Bringen von endogenen Notch-Molekülen mit Notch-Liganden. Der Fachmann kennt Methoden, um endogene Notch-Moleküle mit Notch-Liganden in Kontakt zu bringen, in der Weise, dass der Notch-Signalweg aktiviert wird.

Außerdem bevorzugt ist die Verwendung, wobei Notch-Liganden mit T-Zellen in Kontakt gebracht werden, wobei das In-Kontakt-Bringen durch Stimulation mit Zellen verstärkt und/oder induziert wird, wobei die Zellen ausgewählt sind aus der Gruppe umfassend Antigenpräsentierende Zellen, insbesondere über die Stimulation der Antigen-präsentierenden Zellen mit Toll-like Rezeptoren oder CD40 und/oder plasmazytoiden dendritischen Zellen, insbesondere durch Stimulation der plasmazytoiden dendritischen Zellen über Toll-like Rezeptoren oder CD40.

Dem Fachmann sind Methoden bekannt, um TLR zu aktivieren. Bevorzugt handelt es sich bei den Liganden um Moleküle der Delta-like Familie, insbesondere um Delta-like4 und/oder Delta-like1. Bei den TLR handelt es sich insbesondere um Moleküle, die ausgewählt sind aus der Gruppe umfassend TLR-1, -2, -3, -4, -5, -6, -7, -8, -9, -10, - 11, -12 und/oder -13.

In einer besonders bevorzugten Ausführungsform der Erfindung erfolgt das In-Kontakt-Bringen mit den Notch-Liganden durch Stimulation mit plasmazytoiden dendritischen Zellen, wobei das In-Kontakt-Bringen insbesondere über TLR verstärkt und/oder induziert wird. Plasmazytoide dendritische Zellen exprimieren die Notch-Liganden Delta-like-1 und-4 besonders stark.

In einer weiteren bevorzugten Ausführungsform betrifft die Erfindung die Verwendung von Induktoren der signalaktiven Notch-Moleküle und/oder signalaktive Notch-Moleküle, wobei die Generierung der Interleukin-22 produzierenden T-Zellen in vitro durch eine Stimulation mit Notchligand-Protein, Notchligand-Fusionsproteinen, signalaktiven Notchligand-Fragmenten, Notchligand-exprimierenden Zellen und/oder durch ein Einbringen von signalaktivem Notch durch virale und/oder nicht virale Transduktionsmethoden erfolgt. Die Verwendungen im Sinne der Erfindung führen zu stark erhöhten IL-22-Expressionsraten.

In einer weiteren bevorzugten Ausführungsform betrifft die Erfindung ein in vitro Verfahren zur Generierung von Interleukin-22 produzierenden T-Zellen. Bei dem erfindungsgemäßen Verfahren werden T-Zellen mit signalaktiven Notch-Molekülen in Kontakt gebracht und der Notch-Signalweg somit aktiviert. Das Verfahren ist vorteilhaft, da es besonders einfach und effizient anzuwenden ist. Durch die einfache Umsetzung werden Kosten, Zeit und Arbeitsschritte gespart.

In einer weiteren bevorzugten Ausführungsform betrifft die Erfindung die Verwendung von Inhibitoren der Notch-Aktivierung, ausgewählt aus der Gruppe umfassend rekombinante Proteine, Proteinfragmente und/oder Peptide von Notch oder Notchliganden, pharmakologischen Inhibitoren bevorzugt gamma-Sekretase-Inhibitoren, Antikörpern gegen Notch-Moleküle zur Hemmung der Induktion und/oder Expression von Interleukin-22.

Pharmakologische Inhibitoren des Notch-Signalweges umfassen insbesondere Substanzen der Klasse der gamma-Sekretase-Inhibitoren.

Darüber hinaus sind andere Inhibitoren des Notch-Signalweges einzuschließen, wie lösliche Notch-Liganden, deren Fragmente, Fusionsproteine oder einzelne Peptide wie in der WO2004/024764 offenbart. Weiterhin sind insbesondere natürlich blockierende Antikörper gegen die Notch-Rezeptoren mit beansprucht. Die Inhibition der Notch-Aktivierung ist auch durch die Transfektion der Zellen mit negativen Regulatoren des Notch-Signalweges möglich. Hier sind Deltex, MINT, NRARP oder dominant negative Formen von Mastermind bevorzugte Mittel, die dem Fachmann bekannt sind.

Es war völlig überraschend, dass durch Blockade des Notch-Signalweges die IL-22 Produktion von T-Zellen, bevorzugt proinflammatorischen T-Zellen, verringert oder abgeschaltet wird.

Bevorzugt ist die Verwendung dadurch gekennzeichnet, dass in Antigen-präsentierenden Zellen und/oder dendritischen Zellen die Expression der Notch-Liganden der Delta-like Familie, insbesondere der Liganden Delta-like1 und Delta-like 4, inhibiert ist.

In einer weiteren bevorzugten Ausführungsform betrifft die Erfindung die Verwendung des erfindungsgemäßen Verfahrens zur Induktion von Interleukin-22 in vitro.

Die Erfindung zeichnet sich durch folgende Vorteile aus:
- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff, da aus einer Vielzahl von Möglichkeiten eine bestimmte gewählt wurde, deren Ergebnis nicht vorausgesagt werden konnte, daher handelt es sich um ein patentwürdigen glücklichen Griff)
- Irrtümer in der Fachliteratur bzw. sehr widersprüchliche Darstellung zum Erfindungsgegenstand
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Insbesondere die vorteilhaften Ausführungsformen der Erfindung weisen mindestens einen oder mehrere der genannten Vorteile auf.

### Beispiele

Im Weiteren soll die Erfindung anhand von Beispielen näher erläutert werden, ohne auf diese Beispiele beschränkt zu sein.

### Beispiel 1:

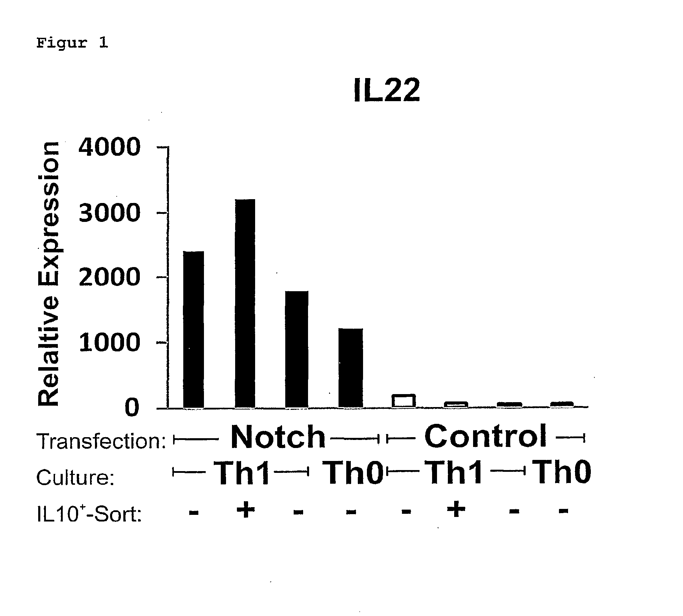

Naive, d.h. Antigen-unerfahrene, T-Helfer-Zellen werden in Gegenwart von Interleukin-12 (TH1) oder ohne IL-12 in vitro aktiviert und mit aktivem Notch retroviral transduziert. Nach 5 Tagen werden die Zellen restimuliert und die IL-22-mRNA Expression analysiert. In einigen Fällen wurden nach der Re-Stimulation IL-10 produzierenden Zellen isoliert. Kontrollzellen produzieren unter diesen Bedingungen kaum IL-22. Notch-transduzierte T-Helferzellen hingegen produzieren große Mengen an IL-22, sowohl unter TH1 als auch unter TH0 Bedingungen. Darüber hinaus wird IL-22 sowohl von IL-10 positiven als auch von IL-10 negativen Th-Zellen produziert (Figur 1).

### Beispiel 2:

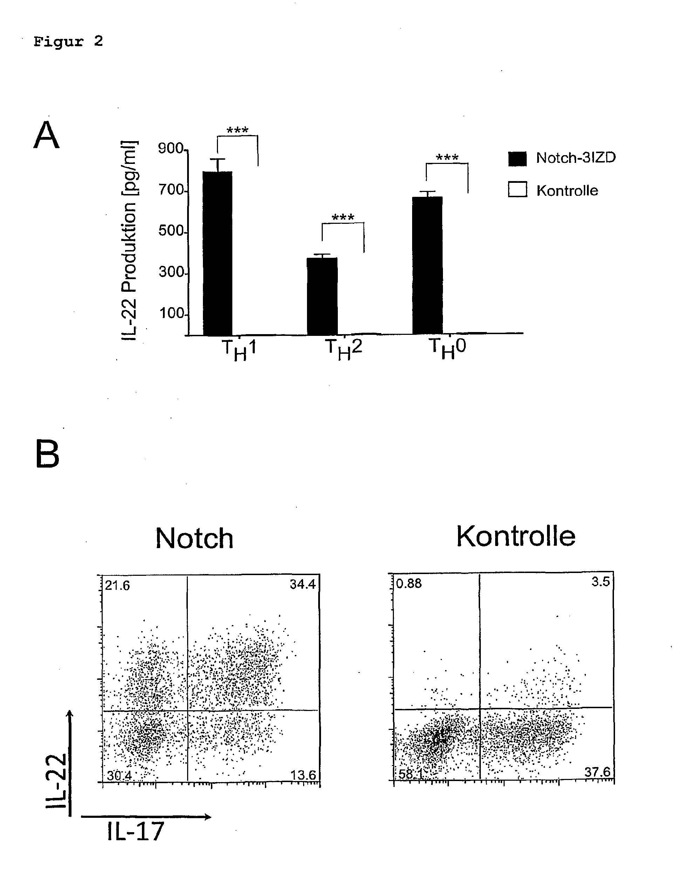

Naive, d.h. Antigen-unerfahrene, T-Helfer-Zellen werden unter TH1-, TH2-, TH0-Bedingungen (Figur 2A) oder TH17-Bedingungen (Figur 2B) in vitro aktiviert und mit aktivem Notch retroviral transduziert. Nach 5 Tagen werden die Zellen restimuliert und die IL-22-Expression analysiert. Im Gegensatz zu kontrolltransduzierten T-Zellen zeigen Notch-transduzierte TH-Zellen unter allen Kulturbedingungen eine starke IL-22 Expression, diese ist in TH17 Zellen besonders ausgeprägt (Figur 2).

### Beispiel 3:

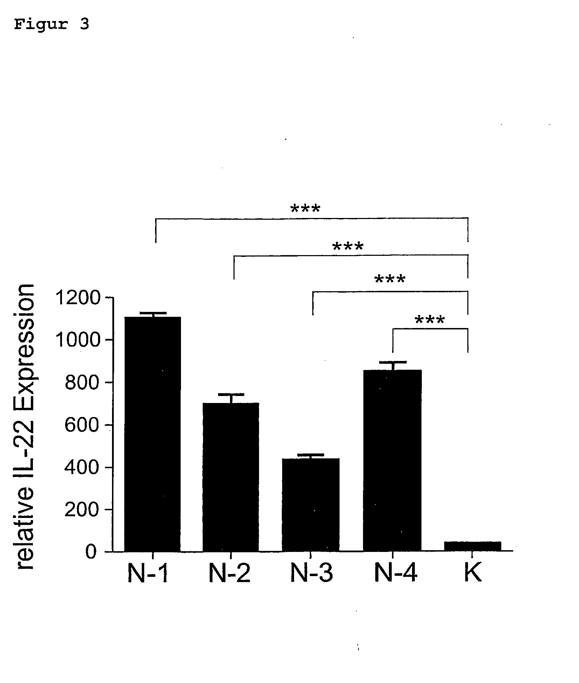

Naive, d.h. Antigen-unerfahrene, T-Helfer-Zellen werden in Gegenwart von Interleukin-12 (TH1) in vitro aktiviert und mit aktivem Notch (Notch-1 bis Notch-4) retroviral transduziert. Nach 5 Tagen werden die Zellen restimuliert und die IL-22-mRNA Expression analysiert. Es zeigt sich, dass alle vier Notch-Isoformen zur Induktion von IL-22 in der Lage sind (Figur 3).

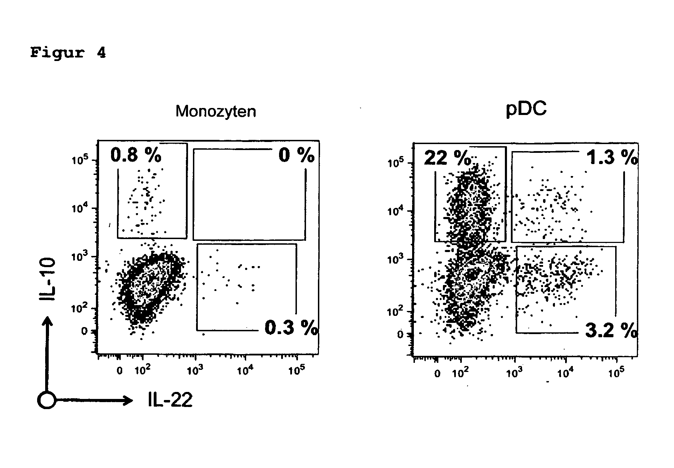

Naive, d.h. Antigen-unerfahrene, humane T-Helfer-Zellen werden mit Monozyten oder mit plasmazytoiden dendritischen Zellen ko-kultiviert und aktiviert. Die pDCs oder Monozyten werden durch den TLR-9 Agonisten CpG aktivert. Nach 5 Tagen werden die Zellen restimuliert und die IL-22 und IL-10 Expression analysiert. Es zeigt sich, dass plasmazytoide dendritische Zellen in der Lage sind, in Th-Zellen die Expression von IL-22 wie auch IL-10 zu induzieren (Figur 4).

### Beispiel 4 :

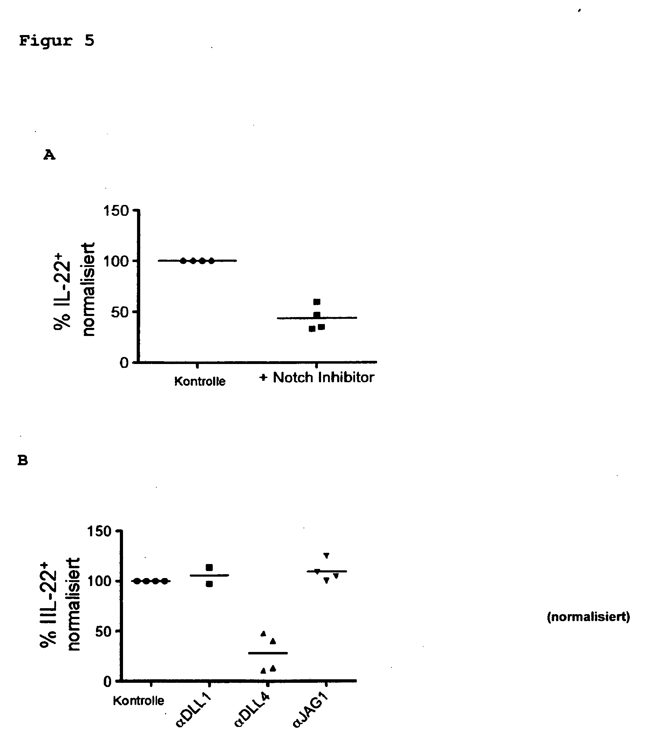

Naive, d.h. Antigen-unerfahrene, humane T-Helfer-Zellen werden mit plasmazytoiden dendritischen Zellen ko-kultiviert und aktiviert. Die pDCs werden durch den TLR-9 Agonisten CpG aktivert. In einigen Ansätzen wird der Notch Siganlweg durch einen spezifischen Inhibitor (g-Sekretase-Inhibitor) inhibiert (Figur 5A). In einigen Ansätzen wird die Interaktion von Notch mit Jagged bzw. mit Dll-1 und Dll-4 selektiv durch blockierende Antikörper inhibiert (Figur 5B). Nach 5 Tagen werden die Zellen restimuliert und die IL-22 Expression analysiert. Es zeigt sich, dass plasmazytoide dendritische Zellen in der Lage sind, in Th-Zellen eine IL-22 Expression zu induzieren. Diese Induktion ist abhängig von der Notch-Aktivität (Figur 4A). Weiterhin ist diese IL-22 Expression abhängig von den Liganden der Delta-like Familie, speziell von Dll-4 (Figur 5B).

### Beispiel 5:

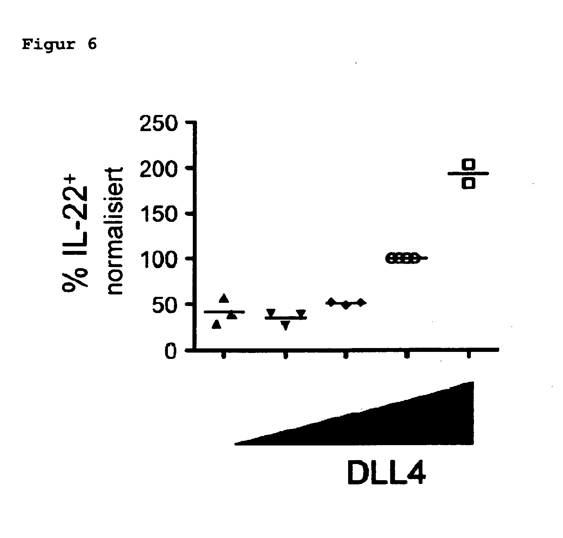

Naive, d.h. Antigen-unerfahrene, humane T-Helfer-Zellen werden in Abwesenheit von antigenpräsentierenden Zellen mit Mikropartikeln, die mit stimulierenden anti-CD3 und anti-CD28 Antikörpern sowie aufsteigenden Konzentrationen von rekombinant hergestelltem Delta-like-4 beladen sind, aktiviert. Nach 5 Tagen werden die Zellen restimuliert und die IL-22 Expression analysiert. Es zeigt sich, dass der Notch Ligand Dll4 konzentrationsabhängig zur Expression von IL-22 führt (Figur 6).

## Patentansprüche

1. In vitro Verwendung von Notch-Regulatoren, ausgewählt aus der Gruppe umfassend Induktoren der signalaktiven Notch-Moleküle und/oder Notch-Inhibitoren, zur Modulation der Interleukin-22-Produktion in T-Zellen.

2. In vitro Verwendung von Induktoren der signalaktiven Notch-Moleküle und/oder signalaktive Notch-Molekülen zur Generierung von Interleukin-22-produzierenden T-Zellen.

3. Verwendung nach Anspruch 1,
**dadurch gekennzeichnet, dass**
die T-Zellen ausgewählt sind aus der Gruppe umfassend naive T-Zellen, Gedächtnis-T-Zellen, proinflammatorische Th1-Zellen, proinflammatorische Th2-Zellen und proinflammatorische Th17-Zellen.

4. Verwendung nach Anspruch 1 oder 2,
**dadurch gekennzeichnet, dass**
die Induktion der signalaktiven Notch-Moleküle erfolgt durch
(i) physiologische Aktivierung der Notch-Rezeptoren, bevorzugt durch Notch-Liganden, Notchligand-Protein, Notchligand-Fusionsproteinen, Notchligand-Fragmente, Notchligand-exprimierenden Zellen und/oder stimulierende anti-Notch-Antikörper und/oder
(ii) Transfektion der T-Zellen mit der konstitutiv aktiven Form von Notch-Rezeptoren.

5. Verwendung nach mindestens einem der vorhergehenden Ansprüche,
**dadurch gekennzeichnet, dass**
die Notch-Moleküle endogene Notch-Moleküle sind.

6. Verwendung nach mindestens einem der vorhergehenden Ansprüche,
**dadurch gekennzeichnet, dass**
die Notch-Rezeptoren ausgewählt sind aus der Gruppe umfassend Notch-1, -2, -3 und/oder -4 und
die Notch-Liganden ausgewählt sind aus der Gruppe umfassend die Jagged-Familie, insbesondere Jagged-1 und/oder -2 und/oder Delta-like-Familie, insbesondere Delta-like-1, -2, und/oder -4.

7. Verwendung nach mindestens einem der vorhergehenden Ansprüche,
**dadurch gekennzeichnet, dass**
signalaktives Notch-1, -2, -3 und/oder -4 überexprimiert wird.

8. Verwendung nach mindestens einem der vorhergehenden Ansprüche,
**dadurch gekennzeichnet, dass**
Notch-Liganden mit T-Zellen in Kontakt gebracht werden, **dadurch gekennzeichnet, dass**
das in-Kontakt-Bringen durch Stimulation mit Zellen verstärkt und/oder induziert wird, wobei die Zellen ausgewählt sind aus der Gruppe umfassend Antigen-präsentierenden Zellen, insbesondere über die Stimulation der Antigen-präsentierenden Zellen mit Toll-like Rezeptoren oder CD40 und/oder plasmazytoiden dendritischen Zellen, insbesondere durch Stimulation der plasmazytoiden dendritischen Zellen über Toll-like Rezeptoren oder CD40.

9. Verwendung nach mindestens einem der vorhergehenden Ansprüche,
**dadurch gekennzeichnet, dass**
die Generierung der Interleukin-22 produzierenden T-Zellen in vitro durch eine Stimulation mit Notchligand-Protein, Notchligand-Fusionsproteinen, signalaktiven Notchligand-Fragmenten, Notchligand-exprimierenden Zellen und/oder durch ein Einbringen von signalaktivem Notch durch virale und/oder nicht virale Transduktionsmethoden erfolgt.

10. In vitro Verwendung von Induktoren der signalaktiven Notch-Moleküle und/oder signalaktiven Notch-Molekülen zur Induktion und/oder Expression von Interleukin-22.

11. In vitro Verfahren zur Generierung von Interleukin-22 produzierenden T-Zellen,
**dadurch gekennzeichnet, dass**
T-Zellen mit signalaktiven Notch-Molekülen in Kontakt gebracht werden und der Notch-Signalweg aktiviert wird.

12. In vitro Verwendung von Inhibitoren der Notch-Aktivierung, ausgewählt aus der Gruppe umfassend rekombinante Proteine, Proteinfragmente und/oder Peptide von Notch oder Notchliganden, pharmakologischen Inhibitoren bevorzugt gamma-Sekretase-Inhibitoren, Antikörpern gegen Notch-Moleküle zur Hemmung der Induktion und/oder Expression von Interleukin-22.

13. Verwendung nach Anspruch 12
**dadurch gekennzeichnet, dass**
in Antigen-präsentierenden Zellen und/oder dendritischen Zellen die Expression der Notch-Liganden der Delta-like Familie, insbesondere der Liganden Delta-like1 und Delta-like 4 inhibiert ist.

## Claims

1. In vitro use of notch regulators selected from the group comprising inductors of the signal-active notch molecule and/or notch inhibitors, for the modulation of interleukin-22 production in T cells.

2. In vitro use of inductors of the signal-active notch molecule and/or signal-active notch molecules for generating interleukin-22 producing T cells.

3. Use according to Claim 1,
**characterized in that**
the T cells are selected from the group comprising naive T cells, memory T cells, proinflammatory Th1 cells, proinflammatory Th2 cells, and proinflammatory Th17 cells.

4. Use according to Claim 1 or 2,
**characterized in that**
the induction of the signal-active notch molecule occurs by
(i) physiological activation of the notch receptors, preferably by notch ligands, notch ligand protein, notch ligand fusion proteins, notch ligand fragments, notch ligand expressing cells and/or stimulating anti-notch antibodies and/or
(ii) transfection of the T cells with the constitutive active form of notch receptors.

5. Use according to at least one of the preceding claims,
**characterized in that**
the notch molecules are endogenous notch molecules.

6. Use according to at least one of the preceding claims,
**characterized in that**
the notch receptors are selected from the group comprising notch-1, -2, -3 and/or -4, and the notch ligands are selected from the group comprising the jagged family, in particular jagged-1 and/or -2 and/or delta-like family, in particular delta-like-1, -2, and/or -4.

7. Use according to at least one of the preceding claims,
**characterized in that**
signal-active notch-1, -2, -3 and/or -4 is overexpressed.

8. Use according to at least one of the preceding claims,
**characterized in that**
notch ligands are brought in contact with T cells,
**characterized in that**
the bringing in contact is reinforced and/or induced by stimulation with cells, wherein the cells are selected from the group comprising antigen-presenting cells, in particular via the stimulation of the antigen-presenting cells with toll-like receptors or CD40 and/or plasmacytoid dendritic cells, in particular by stimulation of the plasmacytoid dendritic cells via toll-like receptors or CD40.

9. Use according to at least one of the preceding claims,
**characterized in that**
the in vitro generation of the interleukin-22 producing T cells in vitro occurs by stimulation with notch ligand protein, notch ligand fusion proteins, signal-active notch ligand fragments, notch ligand expressing cells and/or by introduction of single-active notch using viral and/or nonviral transduction methods.

10. In vitro use of inductors of the signal-active notch molecule and/or signal-active notch molecules for induction and/or expression of interleukin-22.

11. In vitro method for generating interleukin-22 producing T cells,
**characterized in that**
T cells are brought in contact with signal-active notch molecules, and the notch signal pathway is activated.

12. In vitro use of inhibitors of the notch activation, selected from the group comprising recombinant proteins, protein fragments and/or peptides of notch or notch ligands, pharmacological inhibitors preferably gamma-secretase inhibitors, antibodies against notch molecules for the inhibition of the induction and/or expression of interleukin-22.

13. Use according to Claim 12
**characterized in that**
in antigen-presenting cells and/or dendritic cells, the expression of the notch-ligands of the delta-like family, in particular of the ligands delta-like1 and delta-like 4, is inhibited.

## Revendications

1. Utilisation *in vitro* de régulateurs Notch choisis dans l'ensemble comprenant des inducteurs de la molécule Notch activée par signaux et/ou des inhibiteurs Notch, pour la modulation de production d'interleukine-22 dans les cellules T.

2. Utilisation *in vitro* d'inducteurs de la molécule Notch activée par signaux et/ou des molécules Notch activées par signaux pour générer des cellules T produisant de l'interleukine -22.

3. Utilisation selon la revendication 1, **caractérisée en ce que** les cellules T sont choisies dans l'ensemble comprenant des cellules T naïves, des cellules T à mémoire, des cellules Th1 pro-inflammatoires, des cellules Th2 pro-inflammatoires et des cellules Th17 pro-inflammatoires.

4. Utilisation selon la revendication 1 ou 2, **caractérisée en ce que** l'induction de la molécule Notch activée par signaux se produit par
(i) activation physiologique des récepteurs Notch, de préférence par des ligands Notch, par la protéine du ligand Notch, par des protéines de fusion du ligand Notch, par des fragments de ligand Notch, par des cellules d'expression du ligand Notch et/ou par stimulation d'anticorps anti-Notch et/ou
(ii) transfection des cellules T avec la forme active constitutive des récepteurs Notch.

5. Utilisation selon au moins une quelconque des revendications précédentes, **caractérisée en ce que**
les molécules Notch sont des molécules Notch endogènes.

6. Utilisation selon au moins une quelconque des revendications précédentes,
**caractérisée en ce que**
les récepteurs Notch sont choisis dans l'ensemble comprenant Notch-1, -2, -3 et/ou -4, et où les ligands Notch sont choisis dans le groupe comprenant la famille JAG, en particulier JAG-1 et/ou -2 et/ou la famille delta-like, en particulier en delta-like-1, -2 et/ou -4.

7. Utilisation selon au moins une quelconque des revendications précédentes,
**caractérisée en ce que**
le récepteur Notch activé par signaux-1, -2, -3 et/ou -4 est surexprimé.

8. Utilisation selon au moins une quelconque des revendications précédentes,
**caractérisée en ce que**
les ligands Notch sont portés en contact avec les cellules T,
**caractérisée en ce que**
la portée en contact est renforcée et/ou induite par stimulation avec des cellules, les cellules étant choisies dans l'ensemble comprenant des cellules présentant des antigènes, en particulier par la stimulation des cellules présentant des antigènes avec des récepteurs de type Toll (TLR) ou CD40 et/ou des cellules dentritiques plasmacytoïdes, en particulier par stimulation des cellules dentritiques plasmacytoïdes par des récepteurs de type péage ou CD40.

9. Utilisation selon au moins une quelconque des revendications précédentes,
**caractérisée en ce que**
la génération *in vitro* des cellules T produisant de l'interleukine -22 *in vitro* se produit par stimulation avec une protéine de ligand Notch, avec des protéines de fusion de ligand Notch, avec des fragments de ligand Notch activés par signaux, avec des cellules exprimant un ligand Notch et/ou par introduction d'un récepteur Notch à activation simple selon des procédés de transduction virale et/ou non virale.

10. Utilisation *in vitro* d'inducteurs de la molécule Notch activée par signaux et/ou des molécules Notch activées par signaux pour l'induction et/ou l'expression d'interleukine - 22.

11. Procédé *in vitro* servant à produire des cellules T produisant de l'interleukine -22,
**caractérisé en ce que**
les cellules T sont portées en contact avec des molécules Notch activées par signaux, et la voie de signalisation Notch est activée.

12. Utilisation *in vitro* d'inhibiteurs de l'activation Notch, choisis dans l'ensemble comprenant des protéines recombinantes des fragments de protéines et/ou des peptides de récepteurs Notch ou de ligands Notch, des inhibiteurs pharmacologiques, de préférence des inhibiteurs de gamma-sécrétase, des anticorps contre les molécules Notch pour l'inhibition de l'induction et/ou l'expression d'interleukine -22.

13. Utilisation selon la revendication 12, **caractérisée en ce que** les cellules présentant des antigènes et/ou des cellules dentritiques, l'expression des ligands Notch de la famille en delta, en particulier des ligands delta-like 1 et delta-like 4, est inhibée.
